(19)

Europäisches
Patentamt
European
Patent Office
Office européen
des brevets

(11) **EP 2 275 085 A1**

(12) **EUROPEAN PATENT APPLICATION**

(43) Date of publication:
**19.01.2011 Bulletin 2011/03**

(51) Int Cl.:
*A61K 9/00* (2006.01)      *A61K 47/48* (2006.01)
*A61K 48/00* (2006.01)      *A61K 47/34* (2006.01)
*A61K 47/36* (2006.01)

(21) Application number: **09164764.4**

(22) Date of filing: **07.07.2009**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA RS**

(71) Applicant: **University of Rostock
18055 Rostock (DE)**

(72) Inventors:
• **Wang, Weiwei
18057, Rostock (DE)**

• **Li, Wenzhong, Dr.
18069, Rostock (DE)**
• **Ma, Nan, Dr.
18069, Rostock (DE)**
• **Steinhoff, Gustav, Prof.
18211, Rethwisch/Börgerende (DE)**

(74) Representative: **Wablat, Wolfgang
Potsdamer Chaussee 48
14129 Berlin (DE)**

Remarks:
Amended claims in accordance with Rule 137(2)
EPC.

(54) **Biodegradable copolymer suitable for delivering nucleic acid materials into cells**

(57)     The invention is related to a biodegradable copolymer, which is suitable for delivering nucleic acid materials into cells, and which comprises a polysaccharide and at least one cationic polymer or cationic monomer having amino functions, which is bound to the polysaccharide by ester linkage between at least one free hydroxyl group of the polysaccharide and at least one amino group of the cationic polymer or cationic monomer forming an -O-C=O-NH- bond.

The biodegradable copolymer is capable of delivering nucleic acid molecules into the cells in a high efficient way with extremely low cytotoxicity.

**EP 2 275 085 A1**

## Description

The present invention relates to a biodegradable copolymer, which is suitable for delivering nucleic acid materials into cells, comprising a polysaccharide and at least one cationic polymer or cationic monomer having amino functions, which is bound to the polysaccharide by ester linkage between at least one free hydroxyl group of the polysaccharide and at least one amino group of the cationic polymer forming an -O-C=O-NH- bond.

Gene therapy has become an attractive concept for a broad variety of biomedical applications. It provides great opportunities for treating diseases from genetic disorders, infections and cancer. And it has also been considered as a suitable substitute for conventional protein therapy because it can overcome inherent problems during the administration of protein drugs in terms of bioavailability, systemic toxicity, in vivo clearance rate and manufacturing cost [Ledley, F.D., Pharmaceutical approach to somatic gene therapy. Pharmaceutical Research, 1996. 13(11): p. 1595-1614].

The availability of suitable delivery vehicles is the crucial factor for the successful gene therapy. Although viral vectors display rather good transfection properties, such as high transfection efficiency both in vitro and in vivo, there are still many problems associated with these vectors [Gorecki, D.C., Prospects and problems of gene therapy: an update. Expert Opin Emerg Drugs, 2001. 6(2): p. 187-98; McTaggart, S. and M. Al-Rubeai, Retroviral vectors for human gene delivery. Biotechnol Adv, 2002. 20(1): p. 1-31; Zhang, X.J. and W.T. Godbey, Viral vectors for gene delivery in tissue engineering. Advanced Drug Delivery Reviews, 2006. 58(4): p. 515-534], including the immune response against the viral proteins, the possible recombination with wild-type viruses, limitations of the inserted DNA, and difficulties with respect to large-scale pharmaceutical grade production [Lehrman, S., Virus treatment questioned after gene therapy death. Nature, 1999. 401(6753): p. 517-518; Liu, Q. and D.A. Muruve, Molecular basis of the inflammatory response to adenovirus vectors. Gene Therapy, 2003. 10(11): p. 935-940; Sun, J.Y., et al., Immune responses to adeno-associated virus and its recombinant vectors. Gene Therapy, 2003. 10(11): p. 964-976; Donahue, R.E., et al., Helper Virus-Induced T-Cell Lymphoma in Nonhuman-Primates after Retroviral Mediated Gene-Transfer. Journal of Experimental Medicine, 1992. 176(4): p. 1125-1135].

Therefore, recently non-viral gene delivery systems have received a great deal of attention due to the advantages such as they are easy to prepare and have high stability and safety. Cationic lipids and cationic polymers are the two major non-viral vectors that have been widely studied, especially the latter. However, some of the polymers, such as poly(ethylenimine) (PEI), are non-degradable and thus have the risk of accumulation in the body, especially after repeated administration.

In addition, depending on the chemical structure and molecular weigh, most of the cationic polymers show cytotoxicity because of the adverse interactions with the cell membranes, which can induce the loss of cytoplasmic protein, permeabilization of cellular membranes and collapse of the membrane potential [Fischer, D., et al., In vitro cytotoxicity testing of polycations: influence of polymer structure on cell viability and hemolysis. Biomaterials, 2003. 24 (7): p. 1121-1131].

Consequently, one choice is the use of low molecular weight polymers, which can be eliminated via the kidneys. However, the complexes based on the low molecular weight polymers exhibit lower colloidal stability than their high molecular weight counterparts and low transfection efficiencies due to their reduced DNA condensation ability [Fischer, D., et al., In vitro cytotoxicity testing of polycations: influence of polymer structure on cell viability and hemolysis. Biomaterials, 2003. 24(7): p. 1121-1131; Kunath, K., et al., Low-molecular-weight polyethylenimine as a non-viral vector for DNA delivery: comparison of physicochemical properties, transfection efficiency and in vivo distribution with high-molecular-weight polyethylenimine. Journal of Controlled Release, 2003. 89(1): p. 113-125; Ward, C.M., M.L. Read, and L.W. Seymour, Systemic circulation of poly(L-lysine)/DNA vectors is influenced by polycation molecular weight and type of DNA: differential circulation in mice and rats and the implications for human gene therapy. Blood, 2001. 97(8): p. 2221-2229; vandeWetering, P., et al., Relation between transfection efficiency and cytotoxicity of poly(2-(dimethylamino)ethyl methacrylate)/plasmid complexes. Journal of Controlled Release, 1997. 49(1): p. 59-69].

Poly(ethylenimine) (PEI), is one of the most extensively studied cationic polymers, which is widely available, inexpensive and high efficient. It has been used to delivery genes into various cell lines and primary cells both in vitro and in vivo [Li, W.Z., et al., Enhanced thoracic gene detivery by magnetic nanobead-mediated vector. Journal of Gene Medicine, 2008. 10(8): p. 897-909;Li, W.Z., et al., Bc1-2 engineered MSCs inhibited apoptosis and improved heart function. Stem Cells, 2007. 25(8): p. 2118-2127]. PEI with a molecular weight of 25 KDa (25K PEI) showed a high transfection efficiency but also considerable toxicity. Low molecular weight (LMW) PEI is less toxic but shows almost no transfection [Godbey, W.T., K.K. Wu, and A.G. Mikos, Poly(ethylenimine) and its role in gene delivery. Journal of Controlled Release, 1999. 60(2-3): p. 149-160; Fischer, D., et al., A novel non-viral vector for DNA delivery based on low molecular weight, branched polyethylenimine: Effect of molecular weight on transfection efficiency and cytotoxicity. Pharmaceutical Research, 1999. 16(8): p. 1273-1279].

Hence there is a need for biodegradable gene delivery polymers. The expected advantages of such polymers are improved transfection efficiency, reduced toxicity and avoidance of the accumulation of the polymer in the cells. Further it is easier to release DNA into the cytosol due to the degradation of the polymers.

The object of the present invention was the provision of a new compound for delivering nucleic acid materials into cells, which does not have the disadvantages as mentioned above.

The object was achieved by a biodegradable copolymer according to claim 1. In other words, the object was achieved by a biodegradable copolymer, which is suitable for delivering nucleic acid materials into cells, and which comprises a polysaccharide and at least one cationic polymer or cationic monomer having amino functions, which is bound to the polysaccharide by ester linkage between at least one free hydroxyl group of the polysaccharide and at least one amino group of the cationic polymer or cationic monomer forming an -O-C=O-NH- bond. Thus, one could also say that the cationic polymer(s) or monomer(s) are grafted onto the polysaccharide.

This biodegradable copolymer is capable of delivering nucleic acid molecules into the cells in a high efficient way with extremely low cytotoxicity. The cationic polymers or monomers are capable of condensing and delivering nucleic acid materials into cells in a high efficient way with extremely low cytotoxicity. The copolymer has potential applications in gene modification in laboratory research or clinical treatment.

Preferably, the biodegradable copolymer consists of a polysaccharide and at least one cationic polymer or cationic monomer having amino functions, which is bound to the polysaccharide by ester linkage between at least one free hydroxyl group of the polysaccharide and at least one amino group of the cationic polymer or cationic monomer forming an -O-C=O-NH- bond. Overall, one could say that the cationic polymer(s) or monomer(s) are grafted onto the polysaccharide.

It is preferred if the copolymer contains ester bonds that can be hydrolyzed under physiological conditions.

Polysaccharides, which can be used, are amylopectin, glycogen, amylase, glucan, cellulose, maltodextrin, fructan, inulin, mannan, chitin, etc. but amylopectin and glycogen are the preferred ones.

Preferably, the biodegradable copolymer's polysaccharide consists of glucose units, which are linked in a linear way with $\alpha(1{\rightarrow}4)$ bonds and branching via $\alpha(1{\rightarrow}6)$ bonds occurs every 24 to 30 glucose units (amylopectin) or every 8 to 12 glucose units (glycogen).

Amylopectin is a component of starch, supplemented by amylose. Glucose units of amylopectin are linked in a linear way with $\alpha(1{\rightarrow}4)$ bonds and the branching takes place with $\alpha(1{\rightarrow}6)$ bonds occurring every 24 to 30 glucose units. Glycogen is the analogue of plants starch, and is commonly referred to as animal starch. It has the same composition and structure like amylopectin, but with more extensive branching that occurs every 8 to 12 glucose units.

Concerning the polysaccharide, it is preferred if the polysaccharide has a molecular weight of 1.000 to 2.000.000 Dalton, preferably of 10,000 to 1,000,000 Dalton, most preferred of 20,000 to 200,000 Dalton.

By cationic polymers or monomers, it is meant to include polymers or monomers having a molecular weight less than 100.000 Dalton. Preferably, the at least one cationic polymer or cationic monomer having amino functions has a molecular weight of 10 to 100,000 Dalton, preferably of 50 to 50,000 Dalton, most preferred of 500 to 30,000 Dalton. The at least one cationic polymer or cationic monomer having amino functions is preferably selected from the group of polyethylenimine, polylysine, monolysine and derivatives of polyethylenimine, polylysine and monolysine.

Preferably, the biodegradable copolymer has a net positive charge and is capable of containing or complexing with negatively charged nucleic acid molecules such as DNA and RNA and PNA. The net positive is provided by one or more of primary, secondary and tertiary amines that may interact with negatively charged nucleic acid molecules. In other words, the biodegradable copolymer is capable of forming a complex with negatively charged nucleic acids, including DNA, RNA and PNA, all of them single stranded or double stranded, such as plasmid DNA, small interfering RNA (siRNA), and oligonucleotides etc.. The length of the nucleic acid could be from 5 to 2,000,000 base pairs (or bases for single stranded), preferably of 20-25 base pairs for siRNA and 2,000 to 20,000 base pairs for plasmid DNA. Plasmid DNA is preferred.

The bonding between the polysaccharide and at least one cationic polymer or cationic monomer having amino functions can be accomplished in various ways. The preferred method within the scope of the present invention is a method, wherein the polysaccharide is initially reacted with an agent in order to modify or to activate the polysaccharide. This results in a modified or activated polysaccharide, especially a modified or activated amylopectin or glycogen, wherein the modification or activation allows attachment to the at least one cationic polymer or cationic monomer.

A preferred method comprises the steps of:

a) treating the polysaccharide with an agent in order to modify or to activate the polysaccharide resulting in a modified or activated polysaccharide;
b) adding the modified or activated polysaccharide to the at least one cationic polymer or cationic monomer thus forming a mixture and
c) treating the mixture under suitable conditions in order to form the biodegradable copolymer.

Preferably, the agent to modify or to activate the polysaccharide is selected from the group of 1'-carbonyldiimidazole, benzotriazole carbonate, N, N'-disuccinimidyl carbonate, chloroformates, N-hydroxysuccinimidyl chloroformate, and carbonylimidazole, and is preferably 1'-carbonyldiimidazole.

The mixture can be treated under suitable conditions to graft the cationic polymer onto the modified or activated polysaccharide, preferably onto the modified or activated amylopectin or glycogen. The suitable conditions include an almost anhydrous environment, temperatures between -40°C and 150°C, preferably RT and strong stir with magnetic stirrer.

Typically, a polycondensation reaction occurs between the modified or activated polysaccharide, preferably the modified or activated amylopectin or glycogen and the at least one cationic polymer or cationic monomer. Hydroxyl groups of the polysaccharide (amylopectin or glycogen) are coupled to amine groups of the cationic polymer or cationic monomer with the result of a one-carbon spacer.

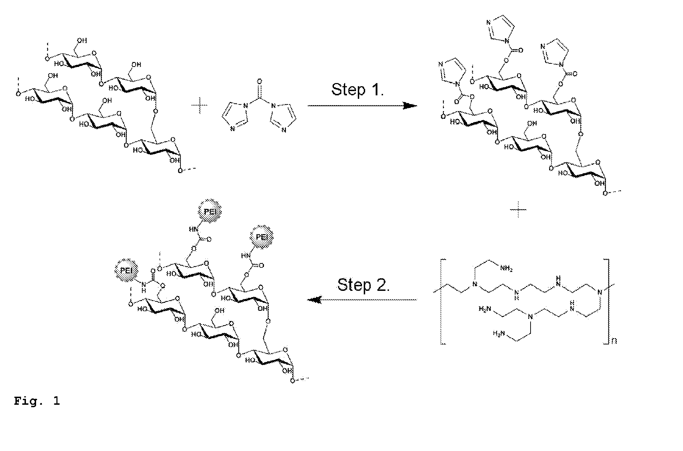

A general scheme of the synthesis, exemplary based on the reaction of Low molecular PEI with a molecular weight 600Da (PEI600), which was grafted onto amylopectin (AMY) via cross-linker 1,1'-carbonyldiimidazole (CDI) to form AMY-PEI600 is represented in Fig. 1.

The degree of bonding between the polysaccharide and the at least one cationic polymer or cationic monomer, i.e. how many free hydroxyl groups of the polysaccharide are bound to amino groups of the cationic polymer or cationic monomer, ranges between 0.0001% and 99%, preferably between 5% and 95%, most preferred between 10% and 90%.

Additionally, the present invention provides a method for delivering a nucleic acid molecule to a cell, wherein the method comprises the formation of a complex between the biodegradable copolymer and a nucleic acid molecule and exposing the cell to the copolymer/nucleic acid molecule complex such that the complex is internalized by the cell and the nucleic acid molecule is released in the cell.

The biodegradable copolymer according to the present invention is used for delivering nucleic acid materials into cells of animals or humans, preferably humans. It is used in the manufacture of a medicament for the delivery of nucleic acid molecule(s) to a cell in an animal. Preferably, the animal is a human. The nucleic acid materials comprise DNA, RNA and PNA, all of them single stranded or double stranded, such as plasmid DNA, small interfering RNA (siRNA), and oligonucleotides etc.. The length of the nucleic acid material could be from 5 to 2000,000 base pairs (or bases for single stranded), preferably of 20-25 base pairs for siRNA and 2,000 to 20,000 base pairs for plasmid DNA. Plasmid DNA is preferred.

The biodegradable copolymer can be diluted or dispersed in any suitable medium for application to cells. Similarly, the copolymers can be formulated in pharmaceutically acceptable diluents for administration to animals including humans. Such formulations are well known in the art. The copolymer can be administered to animals by any suitable route. Typically, the copolymers containing the nucleic acid molecules are administered by injection, typically at the desired site.

In a preferred embodiment, the present invention provides the biodegradable copolymer (AMY-PEI600), synthesized by grafting low molecular weight polyethylenimine (PEI600) onto amylopectin, which was activated by 1,1'-carbonyldiimidazole (CDI). The structure of the copolymer was characterized by FT-IR and the result showed the successful synthesis of this copolymer. The bonding degree was calculated based on the following preconditions:

- the number-average molecular weight (Mn) of polyethylenimine is 600 Dalton;
- in all the amine groups of polyethylenimine, including the primary amine ($-NH_2$), the secondary amine (-NH-) and the tertiary amine (-N=), the percentage of primary amine groups is 25%;
- it was assumed that the bonding point could be only hydroxymethyl groups, but not other hydroxyl groups;
- one polyethylenimine molecular could be bonded to only one hydroxymethyl group.

Based on those preconditions, the bonding degree is 21.97%, i.e. 21.97% mono-saccharide repeating units were bonded by polyethylenimine.

AMY-PEI600 has a net positive charge to bind and condense nucleic acid molecules efficiently. In addition, AMY-PEI600 showed high transfection efficiency which is comparable with the commercial product lipofectin, but has much lower cytotoxicy than PEI25K. This copolymer is biodegradable and has high potential utilization on gene delivery both in vitro and in vivo.

DESCRIPTION OF THE DRAWINGS

FIG.1    Low molecular PEI with a molecular weight 600Da (PEI600) was grafted onto amylopectin (AMY) via cross-linker 1,1'-carbonyldiimidazole (CDI) to form AMY-PEI600.

FIG.2    Fourier transformed infrared spectroscope (FT-IR) of AMY and AMY-PEI600 shows that PEI600 was successfully grafted onto AMY.

FIG.3    DNA retardation assay showed AMY-PEI600 successfully retards DNA completely at N/P ratio (polymer primary nitrogen/DNA phosphate) 0.5, whereas both PEI600 and PEI25K retard DNA completely at N/P ratio 1.0.

FIG.4    AMY-PEI600 successfully condenses DNA to form the complex. The particle size of the complex decreases with the increase of N/P ratio. At lower N/P ratios (2 or 4), AMY-PEI600's DNA condensing ability is much lower than PEI25K's DNA condensing ability; as a result, the particle sizes of AMY-PEI600/DNA are larger. However, at higher N/P ratios (8 and 16), AMY-PEI600 shows similar DNA condensing ability like PEI25K.

FIG.5    Zeta potential assay shows AMY-PEI600/DNA complex has positive charge and the zeta potential value increases with the increase of N/P ratios. PEI25K/DNA complex shows similar tendency but higher zeta potential value at the same N/P ratios. PEI600 doesn't positively charge the DNA.

FIG.6    MTT assay shows that the toxicity of AMY-PEI600, which is similar with PEI600, is much lower than PEI25K at the N/P ratios from 1 to 16. (N=8, * $p \leq 0.01$ and # $p \leq 0.05$ comparing with AMY-PEI600).

FIG.7    Luciferase expression assay shows the best transfection efficiency of AMY-PEI600 is under N/P ratio 8, which is comparable with the commercial product lipofectin (invitrogen) (N=8, * $p \leq 0.01$).

FIG.8    GFP transfection of HEK293 cells with AMY-PEI600 at N/P ratio 8. (A) phase contrast image, (B) GFP positive cells under fluorescent microscope and (C) merged image.

EXAMPLES

**Materials**

Branched polyethylenimine (PEI) with molecular weight (MW) 600Da (PEI600) and 25000Da (PEI25K), amylopectin (from maize), tetrahydrofuran (THF, anhydrous, 99.9%), [3-(4,5-Dimethyl-2-thiazolyl)-2,5-diphenyl-2H-tetrazolium bromide] (MTT), ethidium bromide (EB) and 2,4,6-trinitrobenzenesulfonic acid (TNBS, 5% (w/v) in $H_2O$) were purchased from Sigma-Aldrich (St. Louis, MO, USA). Dimethyl sulfoxide (DMSO, $\geq 99.5\%$) was purchased from ROTH (Roth Chemical, Karlsruhe, Germany). Diethyl ether ($Et_2O$, anhydrous, 99.5%) was purchased from Mallinckrodt Baker (Deventer, Netherlands). 1,1'-Carbonyldiimidazole (CDI) was purchased from Pierce (Rockford, IL, USA). DMSO was dehydrated by distilling once under the reduced pressure before use. Other chemicals and reagents were used directly without further purifications.

**Preparation of AMY-PEI600 copolymer**

Fig. 1 shows the scheme of the AMY-PEI600 copolymer synthesis. Amylopectin and CDI were dissolved in DMSO by stirring under nitrogen at room temperature (RT), respectively. Then, amylopectin solution was added into CDI solution with stirring, followed by an overnight reaction. The resulting AMY-CDI was precipitated followed by drying and re-dissolving in DMSO. PEI600 was dissolved in DMSO by stirring at RT. Then, the AMY-CDI solution was added into PEI600 solution with stirring, followed by an overnight reaction. The final product, AMY-PEI600, was precipitated and washed three times followed by removing the solvent completely at RT. After that, the sticky solid AMY-PEI600 was dissolved in distilled water prior to use. The primary amine concentration of AMY-PEI600 was analyzed according to Snyder SL et. al [Snyder, S.L. and P.Z. Sobocinski, Improved 2,4,6-Trinitrobenzenesulfonic Acid Method for Determination of Amines. Analytical Biochemistry, 1975. 64(1): p. 284-288]. A standard curve was made by a series of PEI600 with known concentration. The primary amine concentration of AMY-PEI600 was quantified via the standard curve.

**Polymer characterization**

Fourier transform infrared (FT-IR) spectroscopy was conducted on a FT-IR spectrometer (Bio-Rad 165, Hercules, CA, USA). Solid samples were ground with KBr and compressed into thin pallets. Sixty four scan signals were averaged with a resolution of $2cm^{-1}$ at room temperature.

**Preparation of plasmid DNA/polymer complex**

Luciferase plasmid (pcDNA3.1-Luc) (Invitrogen, Carlsbad, CA, USA) and green fluorescent protein (GFP) plasmid (pEGFP-N3) (Clonetech, Palo Alto, CA, USA) were used as received without further modification. All plasmids were transformed in *Escherichia coli* DH5α strain and amplified in LB medium at 37°C for overnight at 200 rpm. The amplified plasmid DNA was purified by plasmid DNA purification kit (Macherey-Nagel, Diiren, Germany). The purified plasmid DNA was dissolved in Tris-EDTA (TE) buffer and condensed in a lyophilizer (BETA 1-16, Christ, Osterode, Germany). The concentration and purity of plasmids were determined by ultraviolet (UV) absorbance at 260 and 280 nm with a spectrophotometer (Thermo Electron, Waltham, MA, USA). Finally, the purified plasmid DNA was resuspended in TE buffer and stored in aliquots at a concentration of 2 or 4 mg/ml and stored at -20°C prior to use.

Besides AMY-PEI600, PEI600 and PEI25K were studied for comparison. Briefly, plasmid DNA and polymer were diluted with 5% glucose to the chosen concentration, 0.1mg/ml for DNA and 0.3mM primary amine for polymers. Then, polymer solution was added drop-wise into DNA solution and the mixture was vortexed immediately for 30sec followed by a 30min of incubation at room temperature. The molar ratio of PEI nitrogen in primary amine to DNA phosphate (N/P ratio) was calculated by taking into account that 1$\mu$g DNA contains 3nmol of phosphate and that 43ng PEI (1nmol of $C_2H_5N$ repeat units) holds 0.25nmol of primary amine nitrogen.

### DNA retardation

The retardation of DNA by polymer was studied by gel electrophoresis. Polymer/DNA complex was prepared, mixed with loading buffer and loaded onto an ethidium bromide containing gel (1.5% agarose). Electrophoretic mobility of the samples was measured at room temperature in TBE buffer at 100 V. The DNA bands were visualized using an UV illuminator (Gel Doc 2000 system, Bio-Rad, Hercules, CA, USA).

### Complex size and zeta potential

A dynamic light scattering (DLS) experiment was performed to measure the particle size of the polymer/DNA complex. Complex solution was diluted with 5% glucose to the final DNA concentration of 0.005mg/ml followed by the measurement with Zetasizer Nano ZS (Malvern Instruments, Herrenberg Germany) at 20°C. The refractive index medium (1.332) of 5% glucose at 20°C was used for data analysis. Zeta potential was measured with the same machine, and the measurement was repeated three times for each sample.

### MTT cytotoxicity assay

Human embryonic kidney 293 cell line (HEK293) was cultured in Dulbecco's minimum essential medium (DMEM) supplemented with 10% fetal bovine serum (FBS) and the antibiotics streptomycin (100$\mu$g/ml) and penicillin G (100IU/ml). The cells were grown at 37°C in a humidified atmosphere of air and 5% $CO_2$. For cell viability assay, the cells were seeded into 96-well plate ($0.875\times10^4$cells/well). After 24h of incubation, the culture medium was replaced with fresh medium (175$\mu$l/well), and the polymer/DNA (pcDNA3.1-Luc) complex was added (0.525$\mu$g DNA/well). In parallel, naked DNA and lipofectin (invitrogen, (Invitrogen, Carlsbad, CA, USA)/DNA complex were tested using the same DNA dosage for comparison. The lipofectin/DNA complex was prepared according to the product manual, in serum free DMEM with a lipofectin amount of 6$\mu$l/1$\mu$g DNA. After 24 hours of cells incubation at 37°C, 20$\mu$l MTT (5mg/ml in 1$\times$PBS) was added into each well. And after another 4 hours of incubation at 37°C, the medium was removed and the purple crystals were dissolved in 100$\mu$l dimethylsulfoxide (DMSO). Absorbance was measured at a wavelength of 550nm and a reference wavelength of 655nm using a microplate reader (Model 680, Bio-Rad). The results were expressed as the percentage of viability with respect to the control cells, which were cultured without any treatment. Cell viability was calculated using the equation below:

$$Cell \quad Viability \quad (\%) \quad = \quad (OD_{550}-OD_{655, \; samples} / OD_{550}-OD_{655, \; control}) \times 100\%$$

### Gene transfection assay

The *in vitro* transfection was studied using HEK293 cell line. The cells were seeded into 24-well plate ($0.633\times10^5$cells/well). After 24h of incubation, the culture medium was replaced with fresh medium (1ml/well). The polymer/DNA (pcDNA3.1-Luc) complex, lipofectin/DNA complex and naked DNA was added (3$\mu$g DNA/well). After 40h of transfection, the culture medium was removed. The cells were washed twice by 1$\times$PBS and permeabilized by 200$\mu$l cell lysis buffer (Promega, Madison, WI, USA). The luciferase activity in cell extracts was measured using a luciferase assay kit (Bright-Glo, Promega) on a 96-well microplate luminometer (MicroLumatPlus LB 96V, EG&G Berthold, Bad Wildbad, Germany) for 5s. The relative light units (RLU) were normalized against protein concentration in the cell extracts, which was measured using the BCA protein assay kit (Pierce, Rockford IL, USA) with a microplate reader (Model 680, Bio-Rad).

Based on the optimized parameters by luciferase transfection, the EGFP expression was investigated for confirmation. HEK293 cells were seeded into 24-well plate ($0.5 \times 10^5$cells/well). After 24h of incubation, the culture medium was replaced with fresh medium (1ml/well) and AMY/PEI600/DNA (pEGFP-N3) complex (N/P ratio 8) was added (3μg DNA/well). After 40h of transfection, the cells were observed under fluorescence microscope (Axiovert40 CFL, Carl Zeiss, Goettingen. Germamy).

**Results**

**Fourier transform infrared (FT-IR)**

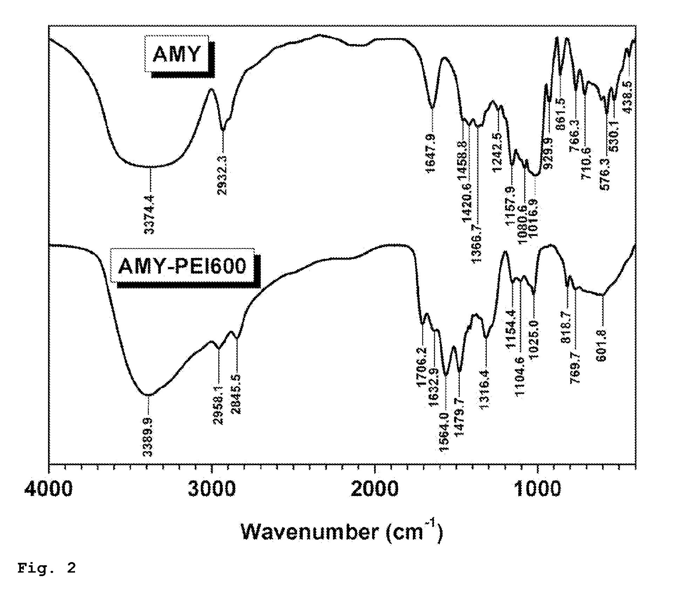

The AMY-PEI600 copolymer was characterized by FT-IR spectroscopy (see Fig. 2). In the spectrum of AMY, a broad band at 3374.4 cm$^{-1}$is probably coming from the symmetric and anti-symmetric O-H stretching vibration. In the spectrum of AMY-PEI600, we can see the peak of N-H stretching vibration (3389.9cm$^{-1}$) overlaid on O-H stretching vibration. In addition, another characteristic peak caused by N-H bending vibration was observed at 1564.0cm$^{-1}$ for AMY-PEI600, which is not found in AMY spectrum. More typically, the carbonyl bonding (C=O) between AMY and PEI600 was observed by the intensive peak at 1706.2cm-1, which is caused by the C=O stretching vibration. After the conjugation of PEI600, the C-H stretching vibration peaks of methyl and methylene were also appeared at 2958.1cm$^{-1}$ and 2845.5cm$^{-1}$. These results showed the PEI600 was successfully conjugated onto the amylopectin.

**Polymer/DNA complex formation**

The formation of polymer/DNA complex was studied by gel electrophoresis, particle size and zeta potential measurements.

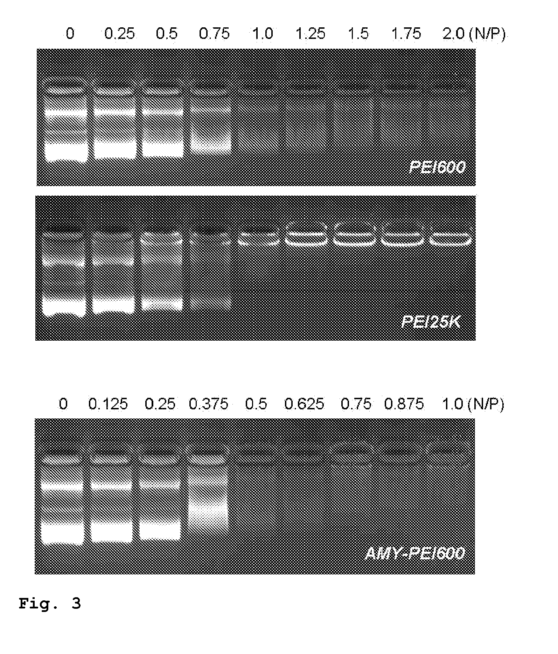

Fig. 3 shows the gel retardation results of PEI600, PEI25K and AMY-PEI600. With the increasing of N/P ratio, all of these three polymers showed increased DNA retardation ability. PEI600 and PEI25K possess the similar DNA retardation capacities. Both of them efficiently complexed and completely retarded DNA at N/P ratio 1.0. However, AMY-PEI600 showed higher DNA retardation capacity, that the DNA was completely retarded at N/P ratio 0.5. Perhaps the reason is the highly branched structure of AMY-PEI600, which makes the DNA be easier to be condensed and retarded.

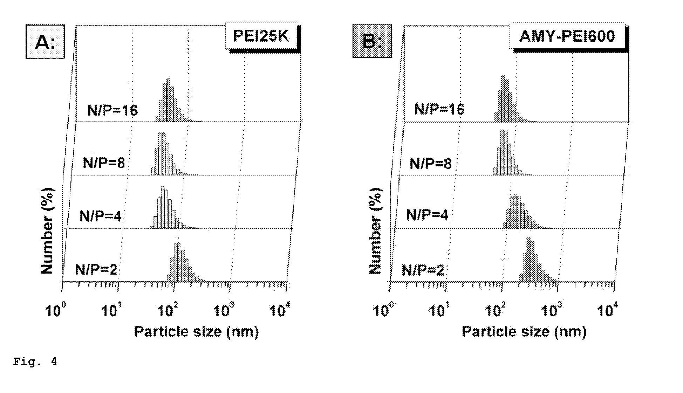

Fig. 4 shows the particle size of AMY-PEI600/DNA complex in comparison with PEI25K/DNA complex at various N/P ratios. Both of PEI25K and AMY-PEI600 could efficiently compact plasmid DNA into small nanoparticles, whereas PEI600 could not compact DNA and the particles are undetectable.

The particle size decreased with the increase of N/P ratio from 2 to 8, for both PEI25K and AMY-PEI600. After N/P ratio 8, the particle size stably remained in a range (30~90nm for PEI25K/DNA, 40~110nm for AMY-PEI600/DNA) until the N/P ratio increases to 16. What should be mentioned is the DNA compact ability of AMY-PEI600 is lower than PEI25K, especially at lower N/P ratio. As a result, at N/P ratio 2 and 4, the particle size of AMY-PEI600/DNA complex (200~900nm at N/P ratio 2 and 90~350nm at N/P ratio 4) is much higher than that of PEI25K/DNA complex (80~250nm at N/P ratio 2 and 30~120nm at N/P ratio 4). Perhaps the reason is due to the difference of the density of primary amine group. Comparing with pure PEI, AMY-PEI has lower amine group density which induces the decrease of the DNA compact ability.

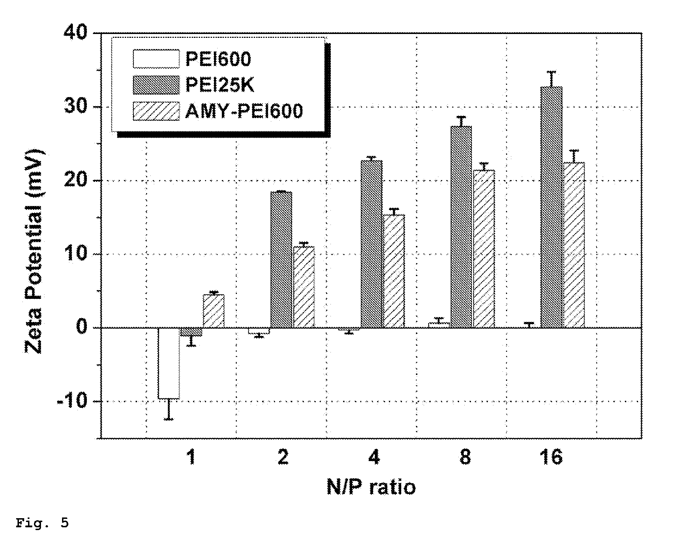

Fig. 5 shows the zeta potential of AMY-PEI600/DNA complex in comparison with PEI600/DNA and PEI25K/DNA complex at various N/P ratios. PEI600 could not compact DNA and positively charge the DNA, but only neutralize the DNA when the N/P ratio is higher than 2. This result confirms the particle size measurement result, in which PEI600 did not show DNA compact capacity. Differently, PEI25K/DNA and AMY-PEI600/DNA complex showed positive surface charge and the zeta potential increased with the increase of N/P ratios. Mostly, except for N/P ratio 1, the zeta potential of PEI25K/DNA is higher than that of AMY-PEI600/DNA. This is perhaps due to the higher DNA compact ability of PEI25K than AMY-PEI600, which has been indicated in particle size analysis.

**Cytotoxicity**

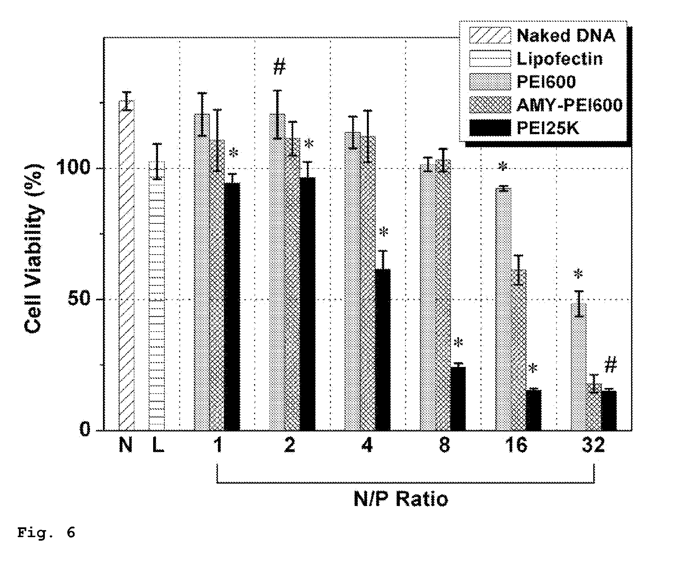

MTT assay was carried out to evaluate the cytotoxicity of AMY-PEI600 using HEK293 cell line. Naked DNA, lipofectin, PEI600 and PEI25K were studied as a reference. The cells that contained only medium without any treatment were used as control, with a cell viability of 100%. The relative viabilities of the treated cells were calculated by comparing with that of control cells. As the result shown in Fig. 6, all of the cytotoxicities of PEI600, PEI25K and AMY-PEI600 increased with the increase of N/P ratio. PEI25K showed very significant toxicity, especially at high N/P ratios. The cell viability decreased very fast form more than 90% (N/P ratio 1 and 2) to about 60% (N/P ratio 4), 20% (N/P ratio 8) and 15% (N/P ratio 16 and 32). In contrast, PEI600 showed low toxicity, though which increased with the increase of N/P ratio. The cells have the viability of more than 90% even at N/P ratio 16. AMY-PEI600 showed the toxicity between PEI25K and PEI600. From N/P ratio 1 to 8, the toxicity of AMY-PEI600 is very low, which allowed more than 100% cell viability and is comparable with PEI600. After that, the cell viability suddenly decreased to about 60% at N/P ratio 16, which is lower than PEI600 but is still much higher than PEI25K. Finally, at N/P ratio 32, the AMY-PEI600 showed similar cell viability (about 17%) like PEI25K, which is significantly lower than PEI600 (about 48%).

**Gene transfection**

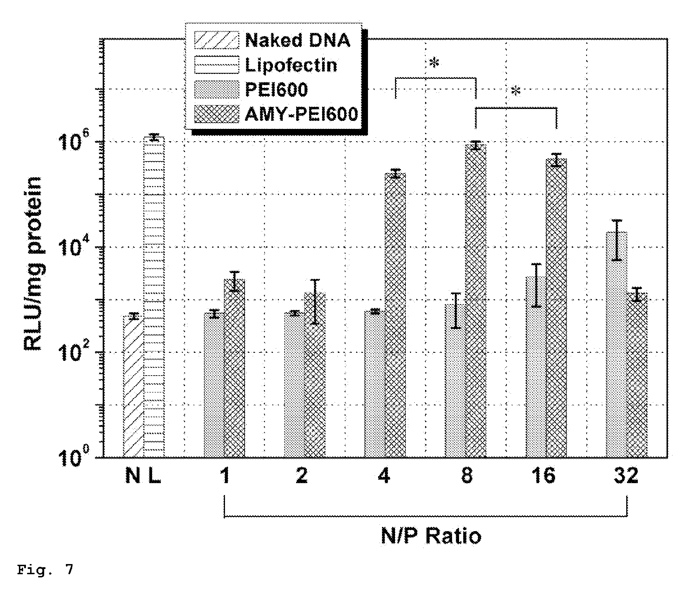

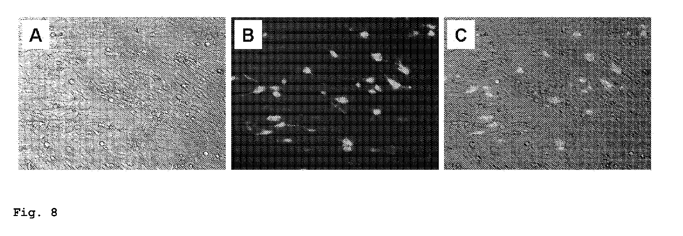

The gene delivery ability of the AMY-PEI600 was tested with HEK293 cell line at the presence of serum. Meanwhile, Naked DNA, lipofectin and PEI600 were tested for comparison. The result was shown in Fig. 7. At lower N/P ratio, PEI600 showed almost no DNA delivery ability. There are no enhancements of the transfection efficiency comparing with naked DNA. With the increase of N/P ratio, the transfection efficiency was improved although it was still low ($2.7 \times 10^3$ and $1.9 \times 10^4$ RLU/mg protein at N/P ratio 16 and 32). AMY-PEI600 showed much higher gene delivery ability than PEI600. The optimized condition is N/P ratio 8, at which it has the comparable transfection efficiency ($0.86 \times 10^6$ RLU/mg protein) with lipofectin ($1.23 \times 10^6$ RLU/mg protein). Fig. 8 showed the EGFP expression in HEK293 cells under the optimized conditions (AMY-PEI600/DNA N/P ratio 8, $3\mu$g DNA/per well of 24-well plate).

**Claims**

1. Biodegradable copolymer, which is suitable for delivering nucleic acid materials into cells, comprising a polysaccharide and at least one cationic polymer or cationic monomer having amino functions, which is bound to the polysaccharide by ester linkage between at least one free hydroxyl group of the polysaccharide and at least one amino group of the cationic polymer or cationic monomer forming an -O-C=O-NH- bond.

2. Biodegradable copolymer according to claim 1 consisting of a polysaccharide and at least one cationic polymer having amino functions, which is bound to the polysaccharide by ester linkage between at least one free hydroxyl group of the polysaccharide and at least one amino group of the cationic polymer or cationic monomer forming an -O-C=O-NH- bond.

3. Biodegradable copolymer according to claim 1 or 2, **characterized in that** the polysaccharide is selected from amylopectin, glycogen, amylase, glucan, cellulose, maltodextrin, fructan, inulin, mannan or chitin.

4. Biodegradable copolymer according to any one of claims 1 to 3, **characterized in that** the polysaccharide consists of glucose units, which are linked in a linear way with $\alpha(1{\to}4)$ bonds and branching via $\alpha(1{\to}6)$ bonds occurs every 24 to 30 glucose units (amylopectin) or every 8 to 12 glucose units (glycogen).

5. Biodegradable copolymer according to any one of claims 1 to 4, **characterized in that** the polysaccharide has a molecular weight of 1,000 to 2,000,000 Dalton, preferably of 10,000 to 1,000,000 Dalton, most preferred of 20,000 to 200,000 Dalton.

6. Biodegradable copolymer according to any one of claims 1 to 5, **characterized in that** the at least one cationic polymer or cationic monomer having amino functions is selected from the group of polyethylenimine, polylysine, monolysine and derivatives of polyethylenimine, polylysine and monolysine.

7. Biodegradable copolymer according to any one of claims 1 to 6, **characterized in that** the at least one cationic polymer or cationic monomer having amino functions has a molecular weight of 10 to 100,000 Dalton, preferably of 50 to 50,000 Dalton, most preferred of 500 to 30,000 Dalton.

8. A method for the preparation of a biodegradable copolymer according to any one of claims 1 to 7, **characterized in that** the polysaccharide is initially reacted with an agent in order to modify or to activate the polysaccharide.

9. Method according to claim 8, comprising the steps of:

   d) treating the polysaccharide with an agent in order to modify or to activate the polysaccharide resulting in a modified or activated polysaccharide;
   e) adding the modified or activated polysaccharide to the at least one cationic polymer or cationic monomer thus forming a mixture and

f) treating the mixture under suitable conditions in order to form the biodegradable copolymer.

**10.** A method for the preparation of a biodegradable copolymer according to claim 8 or 9, **characterized in that** the agent to modify or to activate the polysaccharide is selected from the group of 1'-carbonyldiimidazole, benzotriazole carbonate, N, N'-disuccinimidyl carbonate, chloroformates, N-hydroxysuccinimidyl chloroformate, and carbonylimidazole, and is preferably 1'-carbonyldiimidazole.

**11.** Biodegradable copolymer according to any one of claims 1 to 7 for delivering nucleic acid materials into cells of animals or humans, preferably humans.

**Amended claims in accordance with Rule 137(2) EPC.**

**1.** Biodegradable copolymer, which is suitable for delivering nucleic acid materials into cells, comprising amylopectin or glycogen and at least one cationic polymer or cationic monomer having amino functions, which is bound to the amylopectin or glycogen by ester linkage between at least one free hydroxyl group of the amylopectin or glycogen and at least one amino group of the cationic polymer or cationic monomer forming an -O-C=O-NH- bond.

**2.** Biodegradable copolymer according to claim 1 consisting of amylopectin or glycogen and at least one cationic polymer having amino functions, which is bound to the amylopectin or glycogen by ester linkage between at least one free hydroxyl group of the amylopectin or glycogen and at least one amino group of the cationic polymer or cationic monomer forming an -O-C=O-NH- bond.

**3.** Biodegradable copolymer according to any one of claims 1 to 2, **characterized in that** the amylopectin or glycogen consists of glucose units, which are linked in a linear way with $\alpha(1{\to}4)$ bonds and branching via $\alpha(1{\to}6)$ bonds occurs every 24 to 30 glucose units (amylopectin) or every 8 to 12 glucose units (glycogen).

**4.** Biodegradable copolymer according to any one of claims 1 to 3, **characterized in that** the amylopectin or glycogen has a molecular weight of 1,000 to 2,000,000 Dalton, preferably of 10,000 to 1,000,000 Dalton, most preferred of 20,000 to 200,000 Dalton.

**5.** Biodegradable copolymer according to any one of claims 1 to 4, **characterized in that** the at least one cationic polymer or cationic monomer having amino functions is selected from the group of polyethylenimine, polylysine, monolysine and derivatives of polyethylenimine, polylysine and monolysine.

**6.** Biodegradable copolymer according to any one of claims 1 to 5, **characterized in that** the at least one cationic polymer or cationic monomer having amino functions has a molecular weight of 10 to 100,000 Dalton, preferably of 50 to 50,000 Dalton, most preferred of 500 to 30,000 Dalton.

**7.** A method for the preparation of a biodegradable copolymer according to any one of claims 1 to 6, **characterized in that** the amylopectin or glycogen is initially reacted with an agent in order to modify or to activate the amylopectin or glycogen.

**8.** Method according to claim 7, comprising the steps of:

a) treating the amylopectin or glycogen with an agent in order to modify or to activate the amylopectin or glycogen resulting in a modified or activated amylopectin or glycogen;
b) adding the modified or activated amylopectin or glycogen to the at least one cationic polymer or cationic monomer thus forming a mixture and
c) treating the mixture under suitable conditions in order to form the biodegradable copolymer.

**9.** A method for the preparation of a biodegradable copolymer according to claim 7 or 8, **characterized in that** the agent to modify or to activate the amylopectin or glycogen is selected from the group of 1'-carbonyldiimidazole, benzotriazole carbonate, N, N'-disuccinimidyl carbonate, chloroformates, N-hydroxysuccinimidyl chloroformate, and carbonylimidazole, and is preferably 1'-carbonyldiimidazole.

**10.** Biodegradable copolymer according to any one of claims 1 to 6 is for delivering nucleic acid materials into cells of animals or humans, preferably humans.

Fig. 1

Fig. 2

0    0.25    0.5    0.75    1.0    1.25    1.5    1.75    2.0 (N/P)

0    0.125    0.25    0.375    0.5    0.625    0.75    0.875    1.0 (N/P)

Fig. 3

Fig. 4

Fig. 5

Fig. 6

Fig. 7

Fig. 8

Europäisches
Patentamt

European
Patent Office

Office européen
des brevets

# EUROPEAN SEARCH REPORT

Application Number

EP 09 16 4764

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/102278 A1 (SALAZAR ANDRES [US]; ONCOVIR INC [US]) 3 November 2005 (2005-11-03) * page 2, lines 1-14 * * page 6, line 4 - page 20, line 8 * ----- | 1-11 | INV. A61K9/00 A61K47/48 A61K48/00 A61K47/34 A61K47/36 |
| X | US 2009/087454 A1 (SALAZAR ANDRES [US]) 2 April 2009 (2009-04-02) * paragraphs [0007], [0022], [0036] - [0062] * * claims 1-5 * ----- | 1-11 | |
| X | CHOON KIT TANG, KUO-CHING SHENG, SANDRA E. ESPARON, OWEN PROUDFOOT, VASSO APOSTOLOPOULOS: "Molecular basis of improved immunogenicity in DNA vaccination mediated by a mannan based carrier" BIOMATERIALS, vol. 30, 6 December 2008 (2008-12-06), pages 1389-1400, XP002575810 * abstract * * page 1389, left-hand column - page 1390, left-hand column * * page 1393, left-hand column, line 15 - page 1393, right-hand column, line 3 * ----- | 1-11 | |
| | | | TECHNICAL FIELDS SEARCHED (IPC) |
| | | | A61K |
| X | WO 2008/115799 A1 (UNIV RICE WILLIAM M [US]; HACKER MICHAEL C [US]; SARAF ANITA [US]; MIK) 25 September 2008 (2008-09-25) * claims 1-6 * ----- | 1,2,4-11 | |

The present search report has been drawn up for all claims

| Place of search | Date of completion of the search | Examiner |
|---|---|---|
| Munich | 30 March 2010 | Schifferer, Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 16 4764

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2010

| Patent document cited in search report | | Publication date | Patent family member(s) | | Publication date |
|---|---|---|---|---|---|
| WO 2005102278 | A1 | 03-11-2005 | AU 2003248791 A1<br>EP 1778186 A1 | | 09-11-2005<br>02-05-2007 |
| US 2009087454 | A1 | 02-04-2009 | NONE | | |
| WO 2008115799 | A1 | 25-09-2008 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Ledley, F.D.** Pharmaceutical approach to somatic gene therapy. *Pharmaceutical Research,* 1996, vol. 13 (11), 1595-1614
- **Gorecki, D.C.** Prospects and problems of gene therapy: an update. *Expert Opin Emerg Drugs,* 2001, vol. 6 (2), 187-98
- **McTaggart, S. ; M. Al-Rubeai.** Retroviral vectors for human gene delivery. *Biotechnol Adv,* 2002, vol. 20 (1), 1-31
- **Zhang, X.J. ; W.T. Godbey.** Viral vectors for gene delivery in tissue engineering. *Advanced Drug Delivery Reviews,* 2006, vol. 58 (4), 515-534
- **Lehrman, S.** Virus treatment questioned after gene therapy death. *Nature,* 1999, vol. 401 (6753), 517-518
- **Liu, Q. ; D.A. Muruve.** Molecular basis of the inflammatory response to adenovirus vectors. *Gene Therapy,* 2003, vol. 10 (11), 935-940
- **Sun, J.Y. et al.** Immune responses to adeno-associated virus and its recombinant vectors. *Gene Therapy,* 2003, vol. 10 (11), 964-976
- **Donahue, R.E. et al.** Helper Virus-Induced T-Cell Lymphoma in Nonhuman-Primates after Retroviral Mediated Gene-Transfer. *Journal of Experimental Medicine,* 1992, vol. 176 (4), 1125-1135
- **Fischer, D. et al.** In vitro cytotoxicity testing of polycations: influence of polymer structure on cell viability and hemolysis. *Biomaterials,* 2003, vol. 24 (7), 1121-1131
- **Kunath, K. et al.** Low-molecular-weight polyethylenimine as a non-viral vector for DNA delivery: comparison of physicochemical properties, transfection efficiency and in vivo distribution with high-molecular-weight polyethylenimine. *Journal of Controlled Release,* 2003, vol. 89 (1), 113-125
- **Ward, C.M. ; M.L. Read ; L.W. Seymour.** Systemic circulation of poly(L-lysine)/DNA vectors is influenced by polycation molecular weight and type of DNA: differential circulation in mice and rats and the implications for human gene therapy. *Blood,* 2001, vol. 97 (8), 2221-2229
- **vandeWetering, P. et al.** Relation between transfection efficiency and cytotoxicity of poly(2-(dimethylamino)ethyl methacrylate)/plasmid complexes. *Journal of Controlled Release,* 1997, vol. 49 (1), 59-69
- **Li, W.Z. et al.** Enhanced thoracic gene detivery by magnetic nanobead-mediated vector. *Journal of Gene Medicine,* 2008, vol. 10 (8), 897-909
- **Li, W.Z. et al.** Bc1-2 engineered MSCs inhibited apoptosis and improved heart function. *Stem Cells,* 2007, vol. 25 (8), 2118-2127
- **Godbey, W.T. ; K.K. Wu ; A.G. Mikos.** Poly(ethylenimine) and its role in gene delivery. *Journal of Controlled Release,* 1999, vol. 60 (2-3), 149-160
- **Fischer, D. et al.** A novel non-viral vector for DNA delivery based on low molecular weight, branched polyethylenimine: Effect of molecular weight on transfection efficiency and cytotoxicity. *Pharmaceutical Research,* 1999, vol. 16 (8), 1273-1279
- **Snyder, S.L. ; P.Z. Sobocinski.** Improved 2,4,6-Trinitrobenzenesulfonic Acid Method for Determination of Amines. *Analytical Biochemistry,* 1975, vol. 64 (1), 284-288